# NEUE EUROPÄISCHE PATENTSCHRIFT

(11) **EP 1 638 623 B2**
(45) Veröffentlichungstag und Bekanntmachung des Hinweises auf die Entscheidung über den Einspruch: **28.08.2013**
(45) Hinweis auf die Patenterteilung: 10.03.2010
(21) Anmeldenummer: 04738905.1
(22) Anmeldetag: 30.06.2004
(51) Int. Cl.: A61L 33/00

(54) **MODULARES BESCHICHTUNGSSYSTEM FÜR BIOMATERIALIEN**
MODULAR COATING SYSTEM FOR BIO-MATERIALS
SYSTEME DE REVETEMENT MODULAIRE POUR BIOMATERIAUX

(30) Priorität: 01.07.2003 DE 10330900
(43) Veröffentlichungstag der Anmeldung: 29.03.2006
(73) Patentinhaber: Leibniz-Institut für Polymerforschung Dresden e.V., 01069 Dresden (DE)
(72) Erfinder: GOUZY, Marie-Francoise, 01099 Dresden (DE); WERNER, Carsten, 01069 Dresden (DE); SPERLING, Claudia, 01277 Dresden (DE); SALCHERT, Katrin, 01157 Dresden (DE); STRELLER, Uwe, 01324 Dresden (DE); VOIT, Brigitte, 01187 Dresden (DE); BÖHME, Frank, 01705 Freital-Pesterwitz (DE)
(74) Vertreter: Sperling, Thomas
(86) Internationale Anmeldenummer: PCT/DE2004/001489
(87) Internationale Veröffentlichungsnummer: WO 2005/002640

(56) Entgegenhaltungen:
- WO-A-99/27976
- DE-A- 19 514 104
- DE-A1- 4 115 468
- DE-A1- 19 715 504
- US-A- 5 417 981
- US-A- 5 658 930
- US-A- 5 702 912
- US-A- 6 033 719
- US-A- 6 107 416
- US-A1- 2002 009 491
- US-B1- 6 306 165
- US-B1- 6 486 129
- GOUZY M-F ET AL: "In vitro blood compatibility of polymeric biomaterials through covalent immobilization of an amidine derivative" BIOMATERIALS, ELSEVIER SCIENCE PUBLISHERS BV., BARKING, GB, Bd. 25, Nr. 17, August 2004 (2004-08), Seiten 3493-3501, XP004496076 ISSN: 0142-9612
- GOUZY ET AL.: 'Jahresbericht 2002 des Instituts für Polymerforschung Dresden e.V.', 21 Februar 2003, POLY-DRUCK GMBH, DRESDEN
- UESHIMA ET AL.: 'The effect of argatroban on injured endothelial cells by thrombin' BLOOD COAGULATION AND FIBRIMOLYSIS Bd. 11, Nr. 7, 2000, Seiten 631 - 639
- GOUZY ET AL. POLYMER PREPRINTS: 'Immobilization of small synthetic thrombin inhibitors at the surface of a polymeric biomaterial' Bd. 43, Nr. 2, 2002, Seiten 695 - 696
- POMPE ET AL.: 'Maleic anhydride copolymers-A versatile platform for molecular biosurface engineering' BIOMACROMOLECULES Bd. 4, Nr. 4, 2003, Seiten 1072 - 1079
- STÜRZEBECHER ET AL.: 'Synthetische Inhibitoren der Serinproteinasen' PHARMAZIE Bd. 43, Nr. 11, 1988, Seiten 782 - 783
- HARTMANN ET AL.: 'Funktionalisierung von makromolekularen organischen Verbindungen zur Einführung biologisch aktiver Gruppen' ZEITSCHRIFT FÜR CHEMIE Bd. 27, Nr. 1, Januar 1987, Seiten 1 - 8
- L., STRYER: 'Biochemie', 1994, SPEKTRUM VERLAG Seiten 259 - 260

## Beschreibung

Die Erfindung betrifft ein modulares Beschichtungssystem für Biomaterialien zur Anwendung in Medizinprodukten mit direktem Blutkontakt. Derartige Beschichtungssysteme dienen der Verhinderung der Blutgerinnungsaktivierung durch Biomaterialien, die bei diagnostischen oder therapeutischen Verfahren in Kontakt mit dem Blutkreislauf gebracht werden.

Blutkontaktierende Biomaterialien im Sinne dieser Erfindung werden zum Beispiel für die Herstellung von Medizinprodukten, wie Gefäßprothesen, Herzklappen, implantierbare Blutpumpen, Gefäßstützen und Gefäßkatheter, verwendet. Gleichfalls werden diese Biomaterialien in Blutreinigungssystemen, wie zum Beispiel für Hämodialysemembranen, Oxygenatoren, Schlauchsysteme und Blutbeutel, eingesetzt.

Als Biomaterialien finden häufig Kunststoffe oder Metalle Anwendung. Die Aktivierung der Blutgerinnung und der Thrombusbildung durch die Materialoberflächen stellt beim gegenwärtigen Stand der Technik ein erhebliches Problem für die Sicherheit der Anwendung vieler der oben angeführten Medizinprodukte dar. Die Anwendbarkeit von ansonsten sicheren medizintechnischen Systemen wird dadurch eingeschränkt bzw. es muss anwendungsbegleitend eine zusätzliche Verabreichung von gerinnungshemmenden Medikamenten (Antikoagulantien) vorgenommen werden. Dies bringt jedoch teilweise erhebliche zusätzliche Risiken mit sich.

Zur Verminderung der Blutgerinnung und Thrombusbildung werden daher im Stand der Technik verschiedene Oberflächenmodifikationen und Beschichtungen für blutkontaktierende Biomaterialien vorgeschlagen.
Es wurden physikalische und chemische Oberflächenparameter, wie Texturierung, elektrische Ladung, Hydrophilie und molekulare Beweglichkeit der Materialoberfläche, variiert, womit eine Vermeidung der initialen Aktivierungsreaktionen zwischen Biomaterial und Gerinnungsproteinen bzw. Thrombozyten erreicht werden soll. Außerdem wurden auch bereits verschiedene molekularspezifisch auf das Blutgerinnungssystem oder dessen molekulare Gegenspieler wirkende Biopolymere, wie Heparin, Hirudin oder Thrombomodulin, für Beschichtungen herangezogen. Diese Biopolymere wurden entweder fest an der Materialoberfläche verankert oder als Freisetzungssysteme zeitabhängig vom Material an den Organismus abgegeben.

Im Stand der Technik sind insbesondere Beschichtungen von Biomaterialien mit Heparin bekannt.
Nachteilig ist, dass die spezifische Wirkung von Heparin als Blutgerinnungshemmstoff aufgrund der Strukturvariabilität des Moleküls noch nicht komplett aufgeklärt ist. Weiterhin kann Heparin mit einer Vielzahl von Proteinen in Wechselwirkung treten und so gleichzeitig verschiedene Signalkaskaden beeinflussen.

Nachteilig an einer derartigen Beschichtung ist weiterhin, dass die Wirksamkeit der Beschichtung durch Abbauprozesse, denen diese Moleküle im Organismus unterliegen, zeitlich begrenzt und auch schwer kontrollier- bzw. feststellbar ist. Darüber hinaus weisen Biopolymere, wie Heparin, meist komplexe ausgedehnte dreidimensionale Molekülstrukturen auf, deren Änderung durch die Verbindung mit der Biomaterialoberfläche funktionseinschränkend sein kann.

Im Stand der Technik sind gleichfalls synthetische Blutgerinnungshemmstoffe, wie Serin-Protease-Inhibitoren, bekannt, welche verglichen mit den biomolekularen Vorbildstrukturen kleine molekulare organische Verbindungen darstellen. Diese binden Serin-Proteasen (Thrombin, Faktor Xa) so spezifisch, dass die zur Gerinnung führenden Serinproteasen gehemmt werden.

So wird in der DE 195 14 104 C2 die Nutzung von Blutgerinnungshemmstoffen, auch Inhibitoren genannt, für die Beschichtung von Biomaterialien beschrieben. Dabei erfolgt eine Einlagerung der Inhibitoren in ein bioabbaubares Beschichtungsmaterial, wie etwa Polylactid. In der Anwendung des Biomaterials mit eingelagertem Inhibitor erfolgt dann eine Freisetzung der Inhibitoren aus dem Beschichtungsmaterial.

Nachteilig ist jedoch, dass kleinere, heparinanaloge Substanzen auf Biomaterialoberflächen schwer fixiert werden können, ohne ihre Wirksamkeit stark zu beeinträchtigen. Insbesondere von Nachteil ist, dass in ein Beschichtungsmaterial eingelagerte Inhibitoren gleichfalls zeitabhängig degradiert werden und keine gleichbleibende Aktivität bzw. Hemmung mit derartigen Beschichtungssystemen möglich ist.

Aufgabe der Erfindung ist es, ein Beschichtungssystem für Biomaterialien auszubilden, welches eine spezifische Beeinflussung der Blutgerinnung ermöglicht und gleichzeitig die Wirksamkeit der Blutgerinnungsinhibierung über einen längeren Zeitraum erhalten bleibt.

Erfindungsgemäß wird die Aufgabe durch ein modulares Beschichtungssystem für Biomaterialien zur Anwendung in Medizinkontakten mit direktem Blutkontakt dadurch gelöst, dass eine synthetische Blutgerinnungshemmstoffschicht vorgesehen ist, die über eine flexible Abstandshalterschicht mit der Biomaterialoberfläche verbunden ist, wobei sowohl die Bindung der Abstandshalterschicht mit der Biomaterialoberfläche als auch die Anbindung der Abstandshalterschicht mit den synthetischen Blutgerinnungs-inhibitoren kovalent ausgebildet sind gemäß Anspruch 1 oder 2.

Die Blutgerinnungshemmstoffschicht wird dabei aus einer Substanz mit der Struktur A-B-C ausgebildet, wobei
A eine Guanidino-Gruppe und
B ein alpha-, beta-, para-bi-substituierter Phenylring oder eine Alkylkette ist und C eine Amin- (NH-), Thiol- (S-), Alkohol- (O-) oder eine Carbonsäuregruppe (CO-) ist.

Demnach werden synthetische Inhibitormoleküle wie 4-Guanidino- benzoesäure, ω-Guanidinoalkansäure oder Agmantin durch Umsetzung mit einer primären Amino-, Säure-, Thiol-, oder Alkohol-Gruppe mit einer Spacer- bzw. Abstandhalter-Funktion versehen. Dies erlaubt den Inhibitormolekülen über den Abstandhalter an Oberflächen von Materialien mit komplementären reaktiven chemischen Gruppen kovalent anzuknüpfen und auch im gebundenen Zustand die erforderliche Beweglichkeit zu behalten, um das aktive Zentrum von Gerinnungsenzymen spezifisch binden zu können.

Die Aufgabe der Erfindung wird in alternativer Weise gemäß Anspruch 2 durch eine Blutgerinnungshemmstoffschicht gelöst, die die folgende Struktur aufweist: 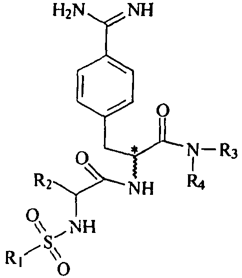 worin
- R₁: ein in der alpha- oder beta-Position gebundener Naphthalinring ist, der gegenbenenfalls mit Alkylgruppen, die bis zu 3 C Atomen enthalten, und/oder Alkoxygruppen mit jeweils bis zu 3 C Atomen, derivatisiert ist, oder ein in der alpha- oder beta-Position gebundener Tetralinring oder Indanring ist, der gegebenenfalls mit Alkylgruppen, die aus bis zu 3 C Atomen bestehen, und oder Alkoxygruppen mit jeweils bis zu 3 C Atomen derivatisiert ist, oder ein Phenylring ist, der gegenbenenfalls mit Alkylgruppen, die bis zu 4 C Atomen enthalten, und/oder mit bis zu drei Gruppen der Struktur O-X, in der O Sauerstoff und X Wasserstoff, Methyl, Ethyl, n-Propyl, i-Propyl oder t-Butyl ist, und/oder mit einer Gruppe der Struktur -COOY, in der Y Wasserstoff, Methyl, Ethyl, n-Propyl, i-Propyl oder t-Buty, i-Butyl, i-Pentyl oder neo-Pentyl ist, derivatisiert ist, oder ein Chromansystem ist, das vorzugsweise mit bis zu 5 Alkylgruppen, die bis zu 3 C Atomen enthalten, derivatisiert ist,
- R₂: eine Gruppe der Struktur D-E ist, wobei 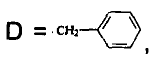 worin der Phenylring in der para-Stellung substituiert ist und E eine NH-, S-, O-, CO-Gruppe ist, und
- R₃ und R₄: gleich oder verschieden sein können und Alkylgruppen mit bis zu 4 Atomen sind oder zusammen einen heterocyclischen Ring mit bis zu 8 Ringgliedern bilden, der weitere Heteroatome enthalten und substituiert sein kann, z. B. der mit einer Hydroxygruppe, Hydroxyalkylgruppe bis zu 3 C Atomen oder Carbonsäuregruppe derivatisiert sein kann,
und in der das mit * gezeichnete C-Atom in der R- und/oder S-Struktur vorliegt.

Derartige Substanzen werden auch als NAPAP-Analoge (N-α-(β-naphthylsulfonyglykol)-4-amidinophenylalanin piperidid) bezeichnet.

Nach dieser alternativen Lösungsmöglichkeit der Aufgabe der Erfindung werden Inhibitormoleküle angeführter Struktur in Verbindung mit Polyethylenglykol-Abstandshaltern verwendet.
Die Abstandshalterschicht im Allgemeinen weist eine Struktur der Form F-G-H auf, wobei die Struktureinheit F eine Amin-, Thiol- oder Alkoholgruppe ist.
Die Struktureinheit G ist eine Alkylkette (CH₂)ₙ mit n= 1 bis 14 oder eine Oligo- bzw. Polyethylenglykolkette im Molekularmassenbereich von 50 bis 10 000 Gramm pro Mol.
Die Struktureinheit H ist eine Amin-, Thiol-, Alkohol- oder eine Carbonsäuregruppe.

Nach der Konzeption der Erfindung wird die Beschichtung durch das Beschichtungssystem derart ausgebildet, dass die Blutgerinnungshemmstoffschicht durch die flexible Abstandshalterschicht sich optimal strukturell ausrichten kann, gleichzeitig aber durch die kovalente Bindung der Abstandshalterschicht mit der Biomaterialoberfläche fest mit dem Biomaterial verbunden ist und weniger stark durch Abbaureaktionen in ihrer Wirksamkeit eingeschränkt wird.

Die Verwendung der angeführten Substanzen als Blutgerinnungshemmstoffschicht ist durch eine spezifischere biologische Aktivität sehr vorteilhaft im Vergleich zu Heparin. Weiterhin kann die physiologische Wirksamkeit aufgrund der synthetischen Kontrolle über die Struktur der Moleküle genau definiert und reproduziert werden.

Die Verknüpfung der abstandshalterfunktionalisierten Inhibitoren mit Oberflächen von Biomaterialien wird vorteilhaft durch die folgenden angeführten Grundprinzipien realisiert.

Die Anknüpfung erfolgt einmal durch die Beschichtung des Materials mit einer Dünnschicht reaktiver Maleinsäureanhydrid-Copolymere, wobei die mit einer solchen Copolymerschicht belegte Materialoberfläche unter spontaner Reaktion mit dem Aminterminus des Polyethlyenglykol-Spacers verbunden wird. Durch dieses Grundprinzip werden verschiedenste Basismaterialien, wie Kunststoffe und Metalle, mit einer reaktiven Basisschicht versehen und so für die kovalente Verankerung von abstandshalterfunktionalisierten synthetischen Blutgerinnungsinhibitoren vorbereitet.

In einer anderen Ausführung können abstandshalterfunktionalisierte synthetische Blutgerinnungsinhibitoren durch Reaktion der Endgruppe der Spacereinheit mit Oberflächenfunktionalitäten von Kunststoffen kovalent immobilisiert werden, die durch Behandlung der Kunststoffe im Niederdruckplasma eingeführt wurden. Ein Beispiel bildet die Reaktion eines amino-terminierten Abstandhalters mit einer säurefunktionalisierten Oberfläche.

Ebenso ist eine zusätzliche Beschichtung mit im Niederdruckplasma behandeltem Polydimethylsiloxan als vorteilhafte Ausgestaltung der Erfindung zu verstehen.

Im Falle einer zusätzlichen reaktiven Polymerbeschichtung der Biomaterialoberfläche wird diese vorteilhaft mit Maleinsäurecopolymer, Polyvinylchlorid, Polyurethane, Polyasparaginsäure, Polyglutaminsäure, Poly(UD-Lysine) oder Poly(hydroxyethylmethacrylat) ausgebildet.

Eine weitere Möglichkeit der festen Bindung der Abstandshalterschicht an das Biomaterial besteht darin, das Biomaterial selbst derart auszuwählen, dass es reaktive Gruppen aufweist. Die Biomaterialoberfläche wird dabei vorteilhaft mit Acrylat-, Acrylamid-, Methacrylat-, Methacrylamid-, Maleimid-, Allyl- oder Vinyl-Gruppen ausgeführt.

Dabei wird konzeptionsgemäß die kovalente Anbindung der Abstandshalterschicht mit den synthetischen Blutgerinnungsinhibitoren dadurch erreicht, dass die Abstandshalterschichtendgruppe mit reakiven Funktionalitäten, wie etwa Maleinsäure-Copolymere, Polyvinylchlorid, Polyurethane, Polyasparaginsäure oder Polyglutaminsäure bei amin-, thiol- und alkoholterminiertem Abstandshalter, versehen wird, wohingegen Poly(UD-Lysine), Poly(hydroxyethylmethacrylat) bei Carbonsäure terminiertem Abstandshalter umgesetzt wird.

Die Erfindung besteht wie erwähnt darin, abstandshalterfunktionalisierte synthetische Blutgerinnungsinhibitoren durch die Reaktion der Endgruppe der Abstandshaltereinheit mit Oberflächenfunktionalitäten von Kunststoffen kovalent zu immobilisieren, wobei die Behandlung der Kunststoffe im Niederdruckplasma erfolgt.

Durch die dargelegten Grundprinzipien können verschiedenste Basismaterialien, wie Kunststoffe oder Metalle, mit einer reaktiven Basisschicht versehen und so für die kovalente Verankerung von abstandshalterfunktionalisierten synthetischen Blutgerinnungsinhibitoren vorbereitet werden.

Die Vorteile der Erfindung liegen in der kovalenten Verankerung synthetischer Blutgerinnungsinhibitoren an der Abstandshalterschicht. Diese Immobilisationsform erweist sich als besonders vorteilhaft, da die feste chemische Verbindung die Freisetzung des Wirkstoffes und damit die zeitabhängige Veränderung der Grenzfläche und den damit verbundenen Verlust des Schutzes gegen die Blutgerinnungsaktivierung und systemische Auswirkungen des freigesetzten Inhibitors wirksam verhindert wird.

In besonderer Weise vorteilhaft ist das modulare Beschichtungssystem nach der Erfindung gegenüber Beschichtungssystemen mit eingebetteten und zeitabhängig freigesetzten Inhibitoren durch die gleichbleibende Aktivität und die konstante lokale Wirkung der Inhibitoren. Hingegen werden die freigesetzten Inhibitoren abgebaut und über den Blutkreislauf von den gewünschten Wirkungsstellen wegtransportiert.

Die Verwendung kleinmolekularer synthetischer Blutgerinnungsinhibitoren in kovalent angebundener Form ist auch deshalb vorteilhaft gegenüber Beschichtungen mit gerinnungsinhibierenden Biopolymeren, da die kovalent gebundene Form bei Verarbeitung, Sterilisation und Lagerung des beschichteten Materials (bzw. Produktes) wesentlich weniger anfälliger für Strukturänderung und Funktionsverlust ist und in der Anwendung aufgrund der kovalenten Bindungen weniger biologischen Abbauprozessen unterliegen.

## Patentansprüche

1. Modulares Beschichtungssystem für Biomaterialien zur Anwendung in Medizinprodukten mit direktem Blutkontakt, aufweisend
• eine synthetische Blutgerinnungshemmstoffschicht, die über
• eine flexible Abstandshalterschicht mit der Biomaterialoberfläche verbunden ist,
• wobei sowohl die Bindung der Abstandshalterschicht mit der Biomaterialoberfläche als auch die Anbindung der Abstandshalterschicht mit den synthetischen Blutgerinnungsinhibitoren kovalent ausgebildet sind und dass die
• Blutgerinnungshemmstoffschicht aus einer Substanz mit der Struktur A-B-C ausgebildet ist, wobei
A eine Guanidino-Gruppe und
B ein alpha-, beta-, para-bi-substituierter Phenylring oder eine Alkylkette ist und
C eine Amin- (NH-), Thiol- (S-), Alkohol- (O-) oder eine Carbonsäuregruppe (CO-) ist, und dass
• die Abstandshalterschicht eine Struktur der Form F-G-H aufweist, wobei
F eine Amin- (NH-), Thiol- (S-), Alkohol- (O-) oder Carbonsäuregruppe (CO-) ist,
G eine Alkylkette (CH₂)ₙ mit n = 1 bis 14 oder eine Oligo- oder Polyethylenglykolkette im Molekularmassenbereich von 200 bis 10.000 g/mol ist und
H eine Amin- (NH-), Thiol- (S-), Alkohol- (O-) oder Carbonsäuregruppe (CO-) ist.

2. Modulares Beschichtungssystem für Biomaterialien zur Anwendung in Medizinprodukten mit direktem Blutkontakt, aufweisend
• eine synthetische Blutgerinnungshemmstoffschicht, die über
• eine flexible Abstandshalterschicht mit der Biomaterialoberfläche verbunden ist,
• wobei sowohl die Bindung der Abstandshalterschicht mit der Biomaterialoberfläche als auch die Anbindung der Abstandshalterschicht mit den synthetischen Blutgerinnungsinhibitoren kovalent ausgebildet sind und dass die
• Blutgerinnungshemmstoffschicht als NAPAP-analoge Substanz ausgebildet ist und folgende Struktur aufweist: 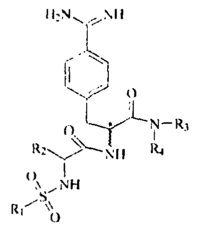 worin
R₁ ausgewählt ist aus:
einem in der alpha- oder beta-Position gebundenen Naphthalinring,
einem in der alpha- oder beta-Position gebundenen Tetralinring oder Indanring,
einem Phenylring oder
einem Chromansystem und
R₂ eine Gruppe der Struktur D-E ist, wobei 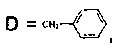 worin der Phenylring in der para-Stellung substituiert ist und E eine Amin- (NH-), Thiol-(S-), Alkohol- (O-) oder Carbonsäuregruppe (CO-) ist und
R₃ und R₄ Alkylgruppen mit bis zu 4 Atomen sind oder zusammen einen heterocyclischen Ring mit bis zu 8 Ringgliedern bilden, der weitere Heteroatome enthalten und substituiert sein kann und der mit einer Hydroxygruppe, Hydroxyalkylgruppe bis zu 3 C Atomen oder Carbonsäuregruppe derivatisiert sein kann,
und in der das mit * gezeichnete C-Atom in der R- und/oder S-Struktur vorliegt und dass
• die Abstandshalterschicht eine Struktur der Form F-G-H aufweist. wobei
F eine Amin- (NH-), Thiol- (S-), Alkohol- (O-) oder Carbonsäuregruppe (CO-) ist,
G eine Alkylkette (CH₂)ₙ mit n = 1 bis 14 oder eine Oligo-bzw. Polyethylenglykolkette im Molekularmassenbereich von 200 bis 10.000 g/mol ist und
H eine Amin- (NH-), Thiol- (S-), Alkohol- (O-) oder Carbonsäuregruppe (CO-) ist.

3. Modulares Beschichtungssystem nach Anspruch 2, **dadurch gekennzeichnet, dass** in R1 der Naphthalinring, der Tetralinring oder der Indanring mit Alkylgruppen, die bis zu 3 C Atome enthalten, und/oder Alkoxygruppen mit jeweils bis zu 3 C Atomen, derivatisiert ist, oder der Phenylring mit Alkylgruppen, die bis zu 4 C Atomen enthalten, und/oder mit bis zu drei Gruppen der Struktur O-X, in der O Sauerstoff und X Wasserstoff, Methyl, Ethyl, n-Propyl, i-Propyl oder t-Butyl ist, und/oder mit einer Gruppe der Struktur - COOY, in der Y Wasserstoff, Methyl, Ethyl, n-Propyl, i-Propyl oder t-Buty, i-Butyl, i-Pentyl oder neo-Pentyl ist, derivatisiert ist, oder das Chromansystem mit bis zu 5 Alkylgruppen, die bis zu 3 C Atomen enthalten, derivatisiert ist.

4. Modulares Beschichtungssystem nach Anspruch 2 oder 3, **dadurch gekennzeichnet, dass** der heterocyclische Ring mit einer Hydroxygruppe, Hydroxyalkylgruppe bis zu 3 C Atomen oder Carbonsäuregruppe derivatisiert ist.

5. Modulares Beschichtungssystem nach einem der Ansprüche 1 bis 4, **dadurch gekennzeichnet, dass** die Biomaterialoberfläche zur Reaktion mit der Abstandshalterschicht
• eine zusätzliche reaktive Polymerbeschichtung aufweist oder
• eine zusätzliche im Niederdruckplasma behandelte Polydimethylsiloxanschicht aufweist oder
• selbst reaktive Gruppen aufweist.

6. Modulares Beschichtungssystem nach Anspruch 5, **dadurch gekennzeichnet, dass** die zusätzliche reaktive Polymerbeschichtung der Biomaterialoberfläche als Maleinsäurecopolymer, Polyvinylchlorid, Polyurethan, Polyasparaginsäure, Polyglutaminsäure, Poly(L/D-Lysine) oder Poly(hydroxyethylmethacrylat) ausgebildet ist.

7. Modulares Beschichtungssystem nach Anspruch 5, **dadurch gekennzeichnet, dass** die Biomaterialoberfläche als reaktive Gruppen Acrylat-, Acrylamid-, Methacrylat-, Methacrylamid-, Maleinimid-, Allyl- oder Vinyl-Gruppen aufweisen.

8. Modulares Beschichtungssystem nach Anspruch 1, **dadurch gekennzeichnet, dass** der Strukturbestandteil A der Blutgerinnungshemmstoffschicht als Guanidino-Gruppe von 4-Guanidinobenzoesäure, ω-Guanidinoalkansäure oder Agmantin ausgebildet ist.

## Claims

1. Modular coating system for biomaterials for use in medical products with direct blood contact, having
• a synthetic clotting inhibitor layer, which
• is connected via a flexible spacer layer with the biomaterial surface,
• wherein both the bond of the spacer layer with the biomaterial layer and also the linking of the spacer layer with the synthetic clotting inhibitors are formed in a covalent manner and that the
• clotting inhibitor layer is formed from a substance with the structure A-B-C, wherein
A is a guanidino group and
B is an alpha, beta, para bi-substituted phenyl ring or an alkyl chain and
C is an amine- (NH-), thiol- (S-), alcohol- (O-) or a carboxylic acid group (CO-) and that
• the spacer layer has a structure of the form F-G-H, wherein
F is an amine- (NH-), thiol- (S-), alcohol- (O-) or carboxylic acid group (CO-),
G is an alkyl chain (CH₂)ₙ with n=1 to 14 or an oligo- or polyethylene glycol chain in the molecular mass range of 200 to 10,000 g/mol and
H is an amine- (NH-), thiol- (S-), alcohol- (O-) or carboxylic acid group (CO-).

2. Modular coating system for biomaterials for use in medical products with direct blood contact, having
• a synthetic clotting inhibitor layer, which
• is connected via a flexible spacer layer with the biomaterial surface,
• wherein both the bond of the spacer layer with the biomaterial surface and also the linking of the spacer layer with the synthetic clotting inhibitors are formed in a covalent manner and that the
• clotting inhibitor layer is formed as a NAPAP-analogous substance and has the following structure: 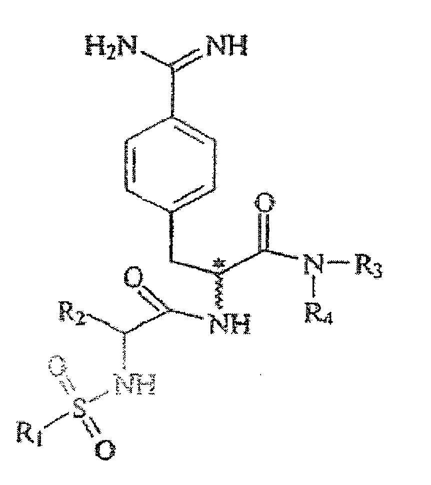 wherein
R₁ is selected from:
a naphthaline ring bonded in the alpha or beta position,
a tetralin ring or indan ring bonded in the alpha or beta position,
a phenyl ring or
a chroman system and
R₂ is a group of the structure D-E, wherein 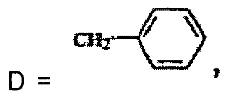 wherein the phenyl ring is substituted in the para position and E is an amine (NH-), thiol (S-), alcohol (O-) or carboxylic acid group (CO-) and
R₃ and R₄ are alkyl groups with up to 4 atoms or together form a heterocyclic ring with up to 8 ring members, which can contain further hetero atoms and be substituted, and which can be derivatized with a hydroxy group, hydroxyalkyl group up to 3 C atoms or carboxylic acid group, and in which the C atom marked with * is present in the R and/or S structure and that
• the spacer layer has a structure of the form F-G-H, wherein
F is an amine (NH-), thiol (S-), alcohol (O-) or carboxylic acid group (CO-),
G is an alkyl chain (CH₂)ₙ with n = 1 to 14 or an oligo- or polyethylene glycol chain in the molecular mass range of 200 to 10,000 g/mol and
H is an amine (NH-), thiol (S-), alcohol (O-) or carboxylic acid group (CO-).

3. Modular coating system according to Claim 2, **characterized in that** in R1 the naphthaline ring, the tetralin ring or the indan ring is derivatized with alkyl groups which contain up to 3 C atoms, and/or alkoxy groups with respectively up to 3 C atoms, or the phenyl ring is derivatized with alkyl groups which contain up to 4 C atoms, and/or with up to three groups of the structure O-X, in which O is oxygen and X is hydrogen, methyl, ethyl, n-propyl, i-propyl or t-butyl, and/or with a group of the structure -COOY, in which Y is hydrogen, methyl, ethyl, n-propyl, i-propyl or t-butyl, i-butyl, i-pentyl or neo-pentyl, or the chroman system is derivatized with up to 5 alkyl groups which contain up to 3 C atoms.

4. Modular coating system according to Claim 2 or 3, **characterized in that** the heterocyclic ring is derivatized with a hydroxy group, hydroxyalkyl group up to 3 C atoms or carboxylic acid group.

5. Modular coating system according to one of Claims 1 to 4, **characterized in that** the biomaterial surface for reaction with the spacer layer
• has an additional reactive polymer coating or
• has an additional polydimethylsiloxane layer treated in the low pressure plasma or
• itself has reactive groups.

6. Modular coating system according to Claim 5, **characterized in that** the additional reactive polymer coating of the biomaterial surface is formed as maleic acid copolymer, polyvinyl chloride, polyurethane, polyaspartic acid, polyglutamic acid, poly(L/D-lysine) or poly(hydroxyethyl methacrylate).

7. Modular coating system according to Claim 5, **characterized in that** the biomaterial surface has as reactive groups acrylate, acrylamide, methacrylate, methacrylamide, maleinimide, allyl or vinyl groups.

8. Modular coating system according to Claim 1, **characterized in that** the structural component A of the clotting inhibitor layer is formed as a guanidino group of 4-guanidinobenzoic acid, w-guanidino-alkane acid or agmantin.

## Revendications

1. Système de revêtement modulaire pour biomatériaux s'utilisant dans les produits médicaux en contact direct avec le sang, présentant
• une couche de matière inhibant la coagulation sanguine qui est reliée par
• une couche d'espacement souple à la surface du biomatériau,
• aussi bien la liaison de la couche d'espacement avec la surface du matériau que le raccordement de la couche d'espacement avec les inhibiteurs synthétiques de coagulation sanguine étant de type covalent,
• la couche de matière inhibant la coagulation sanguine étant constituée d'une substance de structure A-B-C,
A étant un groupe guanidino et
B un anneau de phényle alpha, bêta, para-bi- substitué ou une chaîne alkyle, et
C un groupe amine (NH), thiol (S), alcool (O) ou acide carboxylique (CO) et
• la couche d'espacement présentant une structure de forme F-G-H,
F étant un groupe amine (NH), thiol (S), alcool (O) ou acide carboxylique (CO),
G une chaîne alkyle (CH₂)ₙ, sachant que n = 1 à 14, ou une chaîne oligo- ou polyéthylèneglycol dans la plage de masse moléculaire de 200 à 10 000 g/mole et
H un groupe amine (NH), thiol (S), alcool (O) ou acide carboxylique (CO).

2. Système de revêtement modulaire pour biomatériaux s'utilisant dans les produits médicaux en contact direct avec le sang, présentant
• une couche de matière inhibant la coagulation sanguine qui est reliée par
• une couche d'espacement souple à la surface du biomatériau,
• aussi bien la liaison de la couche d'espacement avec la surface du biomatériau que le de type covalent avec les inhibiteurs synthétiques de coagulation sanguine raccordement de la couche d'espacement étant , et
• la couche de matière inhibant la coagulation se présentant sous forme de substance analogue à NAPAP et présentant la structure suivante : 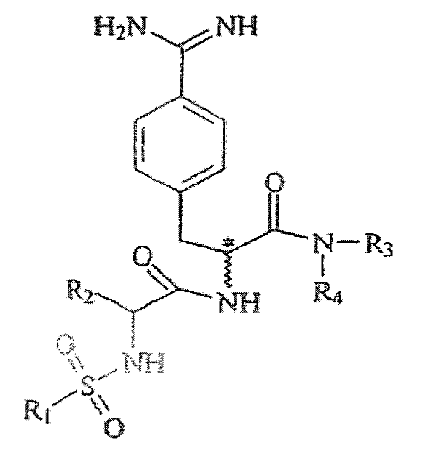 sachant que
R₁ est sélectionné parmi :
un anneau de naphtaline lié dans la position alpha ou bêta,
un anneau de tétraline ou d'indane lié dans la position alpha ou bêta,
un anneau de phényle ou
un système de chromane et
R₂ est un groupe de structure D-E, sachant que 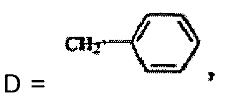 l'anneau de phényle étant substitué dans la position para et E étant un groupe amine (NH), thiol (S), alcool (O) ou acide carboxylique (CO) et que
R₃ et R₄ sont des groupes alkyle comportant jusqu'à 4 atomes ou forment ensemble un anneau hétérocyclique comportant jusqu'à 8 éléments d'anneau et pouvant contenir d'autres hétéroatomes et être substitué et pouvant être dérivatisé avec un groupe hydroxy, un groupe hydroxyalkyle jusqu'à 3 atomes de C ou un groupe acide carboxylique, et dans lequel l'atome de C désigné par * est présent dans la structure R et/ou S et
• la couche de matière inhibant la coagulation présentant une structure de forme F-G-H,
F étant un groupe amine (NH), thiol (S), alcool (O) ou acide carboxylique (CO),
G une chaîne alkyle (CH₂)ₙ, sachant que n = 1 à 14, ou une chaîne oligo- ou polyéthylèneglycol dans la plage de masse moléculaire de 200 à 10 000 g/mole et
H un groupe amine (NH), thiol (S), alcool (O) ou acide carboxylique (CO).

3. Système de revêtement modulaire selon la revendication 2, **caractérisé en ce que**, dans R1, l'anneau de naphtaline, l'anneau de tétraline ou l'anneau d'indane est dérivatisé avec des groupes alkyle contenant jusqu'à 3 atomes de C et/ou des groupes alkoxy contenant respectivement jusqu'à 3 atomes de C ou que l'anneau de phényle est dérivatisé avec des groupes alkyle contenant jusqu'à 4 atomes de C et/ou avec jusqu'à trois groupes de structure O-X dans laquelle O est de l'oxygène et X de l'hydrogène, du méthyle, de l'éthyle, du n-propyle, de l'i-propyle ou du t-butyle et/ou avec un groupe de structure -COOY dans lequel Y est de l'hydrogène, du méthyle, de l'éthyle, du n-propyle, de l'i-propyle ou du t-butyle, de l'i-butyle, de l'i-pentyle ou du néopentyle ou le système de chromane avec jusqu'à 5 groupes alkyle contenant jusqu'à 3 atomes de C.

4. Système de revêtement modulaire selon la revendication 2 ou 3, **caractérisé en ce que** l'anneau hétérocyclique est dérivatisé avec un groupe hydroxy, un groupe hydroxyalkyle jusqu'à 3 atomes de C ou un groupe acide carboxylique.

5. Système de revêtement modulaire selon une des revendications 1 à 4, **caractérisé en ce que** la surface du biomatériau présente, pour la réaction avec la couche d'espacement,
• un revêtement polymérique réactif supplémentaire ou
• une couche supplémentaire de polydiméthylesiloxane traitée sous plasma à basse pression ou
• présente des groupes autoréactifs.

6. Système de revêtement modulaire selon la revendication 5, **caractérisé en ce que** le revêtement polymérique réactif supplémentaire de la surface de biomatériau se présente sous forme d'un copolymère d'acide maléique, d'un chlorure de polyvinyle, d'un polyuréthane, de polyasparagine, d'acide polyglutaminique, de poly(L/D-lysine) ou de poly(hydroéthyleméthacrylate).

7. Système de revêtement modulaire selon la revendication 5, **caractérisé en ce que** la surface du biomatériau présente comme groupes réactifs des groupes acrylate, acrylamide, méthacrylate, méthacrylamide, maléinimide, allyle ou vinyle.

8. Système de revêtement modulaire selon la revendication 1, **caractérisé en ce que** le composant structurel A de la couche de matière inhibant la coagulation du sang se présente sous forme de groupe guanidino de 4-acide guanidinobenzoïque, ω-acide quadininoalkanique ou d'agmantine.
